Europäisches Patentamt

European Patent Office

Office européen des brevets

(11) Veröffentlichungsnummer : **0 040 799**
**B1**

# (12) EUROPÄISCHE PATENTSCHRIFT

(45) Veröffentlichungstag der Patentschrift :
26.10.83

(21) Anmeldenummer : 81103849.6

(22) Anmeldetag : 19.05.81

(51) Int. Cl.³ : **C 12 N   9/74,** C 12 N   9/96,
C 12 Q   1/56

(54) Stabilisiertes Thrombinpräparat.

(30) Priorität : 22.05.80 DE 3019612

(43) Veröffentlichungstag der Anmeldung :
02.12.81 Patentblatt 81/48

(45) Bekanntmachung des Hinweises auf die Patenterteilung : 26.10.83 Patentblatt 83/43

(84) Benannte Vertragsstaaten :
AT BE CH DE FR GB IT LI LU NL SE

(56) Entgegenhaltungen :
EP A 0 004 271
DE A 2 807 089
FR A 2 193 029
FR A 2 369 291

CHEMICAL ABSTRACTS, Band 70, Nr. 13, 31.
März 1969, Seite 44, Nr. 54331x Columbus, Ohio,
U.S.A. G.F. LANCHANTIN et al. : « Interaction of
soybean trypsin Inhibitor with thrombin and its
effect on prothrombin activation »

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : Lill, Helmut, Dr.
Zugspitzstrasse 24
D-8121 Wielenbach (DE)
Erfinder : Bartl, Knut, Dr.
Am Westend 6
D-8121 Wilzhofen (DE)

(74) Vertreter : Weickmann, Heinrich, Dipl.-Ing. et al
Patentanwälte Dipl.-Ing. H. Weickmann Dipl.-Phys.Dr.
K. Fincke Dipl.-Ing. F.A. Weickmann Dipl.-Chem. B.
Huber Dr.-Ing. H. Liska Möhlstrasse 22
D-8000 München 86 (DE)

Stabilisiertes Thrombinpräparat

Die Erfindung betrifft ein stabilisiertes Thrombinpräparat in fester oder gelöster Form, welches insbesondere für diagnostische Zwecke geeignet ist.

Thrombin (Blutgerinnungsfaktor IIa) spielt eine wichtige Rolle im Blutgerinnungssystem. Es wirkt einerseits zurück auf das Prothrombin (Faktor II) im Sinne einer Regulation des Gerinnungsvorgangs, vor allem aber bewirkt es die Überführung von Fibrinogen (Faktor I) in Fibrinmonomer (FS) unter Peptidabspaltung, welches dann zum Fibrinpolymer gerinnen kann. Thrombin spielt daher vor allem in der Diagnostik des Blutgerinnungssystems eine wichtige Rolle. Beispielsweise wird es zur Bestimmung von Heparin oder von Antithrombin III verwendet.

Die Verwendung des Thrombins für Untersuchungen des Blutgerinnungssystems, insbesondere in Testsystemen, die für diesen Zweck bestimmt sind, wird durch die außerordentlich große Instabilität des Thrombins stark beeinträchtigt. Insbesondere in gelöster Form ist die Instabilität sehr ausgeprägt. Beispielsweise ist eine wäßrige Thrombinlösung bei 0 °C nur ca. 5 Stunden stabil (Thromb. Diath. Hemorrh. 9, 169 (1963)). Für manche Präparate wird in gelöster Form bei 0 °C eine Stabilität von nur 1 Stunde angegeben.

Abgesehen von dieser allgemeinen Instabilität von Thrombin wurde gefunden, daß gelegentlich außerordentlich rasch sehr starke Aktivitätsveränderungen im Sinne einer Inaktivierung bei Thrombin auftreten, die bei Verwendung im Rahmen analytischer Tests zu völlig unbrauchbaren Resultaten führen. Versuche haben Anhaltspunkte dafür ergeben, daß diese Aktivitätsveränderung durch Behälteroberflächen bedingt ist, mit welchen die Thrombinlösung in Berührung kommt.

Der Erfindung liegt daher die Aufgabe zugrunde, diese mit der Verwendung von Thrombin zusammenhängenden Schwierigkeiten zu beseitigen und ein stabilisiertes Thrombinpräparat zu entwickeln, welches sowohl in fester als auch in gelöster Form ein für praktische Zwecke ausreichende Gebrauchsdauer aufweist und bei dem insbesondere auch unvorhersehbare zeitunabhängige Aktivitätsänderungen vermieden werden.

Gelöst wird diese Aufgabe erfindungsgemäß durch ein stabilisiertes Thrombinpräparat in fester oder gelöster Form, insbesondere für analytische Zwecke, welches dadurch gekennzeichnet ist, daß es als Stabilisatoren Serumalbumin in Kombination mit mindestens einem Proteasenhemmer und mindestens einem Chelatbildner enthält.

Die Erfindung beruht auf der Feststellung, daß die drei Komponenten des erfindungsgemäßen Mittels eine synergistische Wirksamkeit ausüben, die zu einem sehr stabilen Thrombinpräparat führt, bei welchem auch die bisher beobachteten Ausreißer im Sinne einer plötzlichen Aktivitätsabnahme nicht auftreten. So weist ein erfindungsgemäß stabilisiertes Thrombonpräparat in Form einer wäßrigen Lösung bei einmonatiger Lagerung bei 33 °C noch eine Aktivität von etwa 80 % des Ausgangswerts auf. Wird eine wäßrige Lösung des erfindungsgemäßen Präparats in üblicher Weise bei etwa 0 °C gelagert, so weist sie viele Monate lang eine nur unwesentlich veränderte Aktivität auf.

Vorzugsweise enthält das erfindungsgemäß stabilisierte Thrombinpräparat Chelatbildner in einer Menge zwischen 0,01 und 0,2 M, Proteasenhemmer in einer Menge von 1 bis 100 µg/ml und Serumalbumin in einer Menge von 5 bis 100 mg/ml bzw., soweit es sich um ein Trockenpräparat handelt, in einer Menge, die bei Auflösen in der vorgesehenen Lösungsmittelmenge derartige Konzentrationen ergibt.

Unter den Chelatbildnern werden die sechszähnigen Liganden bevorzugt. Beispiele hierfür sind Nitrilotriessigsäure, Cyclohexylen-(1,2)-dinitrilotetraessigsäure, Diäthylentriamin-pentaessigsäure und Äthylendiaminotetraessigsäure (EDTA). Bevorzugt wird EDTA. Die besten Ergebnisse werden erhalten, wenn der Chelatbildner in einer Konzentration von 0,03 bis 0,1 M vorliegt.

Als Proteasenhemmer kommen im erfindungsgemäßen, stabilisierten Thrombinpräparat solche in Betracht, die Thrombin selbst nicht hemmen. Beispiele hierfür sind Aprotinin und Sojabohnen-trypsininhibitor (STi). Weitere geeignete Proteaseinhibitoren finden sich in « The Enzymes », Vol. III, 3. Auflage 1971, S. 380-382 angegeben. Bevorzugt wird Aprotinin verwendet, da es als Handelspräparat leicht zugänglich ist.

Die dritte Komponente des im erfindungsgemäßen Thrombinpräparat enthaltenen Stabilisierungsmittels ist Serumalbumin. Das Serumalbumin kann beliebiger Herkunft sein, bevorzugt wird jedoch Rinderserumalbumin (RSA) aufgrund seiner leichten Zugänglichkeit. Wie bereits erwähnt, sollte die Lösung 0,5 bis 10 %, vorzugsweise 1 bis 5 % (10 bis 50 mg/ml) enthalten. Nach den vorliegenden Resultaten scheint vor allem das Serumalbumin den plötzlichen Aktivitätsabfall von Thrombin zu verhindern. Es wird vermutet, daß der erwähnte Aktivitätsabfall durch Detergentienspuren, insbesondere durch anionische Detergentienspuren, die bei der Reinigung von Geräten und Gefäßen, mit denen die Thrombinlösung in Berührung kommt, zurückgeblieben sind, verursacht werden.

Das erfindungsgemäße, stabilisierte Thrombinpräparat kann als solches oder im Gemisch mit anderen Substanzen in Form eines kombinierten Reagensgemisches vorliegen. Wenn es in gelöster Form vorliegt, so soll ein etwa neutraler pH-Wert (6,0 bis 8,0, vorzugsweise 6,5 bis 7,5) vorliegen. In den meisten Fällen reicht die Pufferkapazität des Chelatbildners aus, um diesen pH-Bereich einzustellen. Jedoch kann auch eine übliche Puffersubstanz zugesetzt werden. Auch das Trockenpräparat, welches im allgemeinen in lyophilisierter Form vorliegt, kann eine solche Puffersubstanz bereits enthalten.

# 0 040 799

Bei handelsüblichen Thrombinpräparaten nimmt im Durchschnitt die Aktivität bei Lagerung in wäßriger Lösung bei Raumtemperatur in zwei Wochen um etwa 50 % ab. Unter den gleichen Bedingungen tritt beim erfindungsgemäß stabilisierten Präparat im Rahmen der Fehlergrenze keine Aktivitätsabnahme auf.

Die folgenden Beispiele erläutern die Erfindung.

## Beispiel 1

117 mg eines stabilisierten Thrombin-Lyophilisats der Zusammensetzung :
47,9 % EDTA, 51,28 % RSA, 0,02 % Aprotinin, 0,5 % Natriumazid (als Konservierungsmittel), 0,3 % Thrombin werden mit 3 ml bidestilliertem Wasser gelöst und die Stabilität der Lösung untersucht.
Stabilität der Thrombinlösung :

| | Thrombinaktivität (pH 8,1 ; 25 °C ; TOS-Gly-Pro-Arg-pNa als Substrat | | |
|---|---|---|---|
| | Ausgangs-Wert (U/ml) | 1 Woche (U/ml) | 2 Wochen (U/ml) |
| Lyophilisat bei + 4 °C gelagert. Lösung bei + 4 °C gelagert | 0,46 | 0,46 | 0,48 |
| Lyophilisat bei + 4 °C gelagert. Lösung bei Raumtemperatur gelagert | 0,47 | 0,46 | 0,48 |
| Lyophilisat 3 Wochen bei 35 °C gelagert. Lösung bei + 4 °C gelagert | 0,46 | 0,46 | 0,47 |
| Lyophilisat 3 Wochen bei 35 °C gelagert. Lösung bei Raumtemperatur gelagert | 0,47 | 0,46 | 0,47 |

## Beispiel 2

Die stabilisierende Wirksamkeit des erfindungsgemäßen stabilisierten Thrombinpräparats wurde verglichen mit der stabilisierenden Wirksamkeit der Einzelkomponenten und der verschiedenen möglichen Kombinationen von jeweils zwei der drei erfindungsgemäß vorhandenen Komponenten.

In der nachstehenden Tabelle wird die Abnahme der Thrombinaktivität in Prozent bei den verschiedenen stabilisierten Thrombinpräparaten angegeben. Die EDTA-Menge betrug 50 mM, pH 6,9 ; die Aprotininmenge betrug 1 mg/100 ml, die RSA-Menge 2 g/100 ml.

## Tabelle

Einfluß von EDTA, Aprotinin und Serumalbumin auf die Thrombin-Stabilität

| Lagerung bei 35 °C (Wochen) | Bid. Wasser | RSA | Aprotinin | Aprotinin RSA | EDTA | EDTA Aprotinin | EDTA RSA | EDTA Aprotinin RSA |
|---|---|---|---|---|---|---|---|---|
| 1 | 87,5 | 85,7 | 65,8 | 82,2 | 17,0 | 10,6 | 10,0 | 8,3 |
| 2 | 98,0 | 98,0 | 89,5 | 95,6 | 31,9 | 12,8 | 12,0 | 10,4 |

Aus den obigen Werten ergibt sich, daß Serumalbumin keinen und der Proteasenhemmer nur einen sehr geringen stabilisierenden Einfluß ausüben und die Kombination Serumalbumin/Proteasenhemmer ebenfalls überhaupt nicht stabilisiert. Der Chelatbildner stabilisiert zwar, jedoch wird durch Zusatz der für sich allein völlig unwirksamen Mischung von Serumalbumin und Proteasenhemmer eine weitaus überlegene Stabilisierungswirkung erzielt.

## Ansprüche

1. Stabilisiertes Thrombinpräparat in fester oder gelöster Form, insbesondere für analytische Zwecke, dadurch gekennzeichnet, daß es als Stabilisatoren Serumalbumin in Kombination mit mindestens einem Proteasenhemmer und mindestens einem Chelatbildner enthält.

0 040 799

2. Stabilisiertes Thrombinpräparat nach Anspruch 1, dadurch gekennzeichnet, daß es

0,01 bis 0,2 M Chelatbildner,
1 bis 100 µg/ml Proteasenhemmer und

5 bis 100 mg/ml Serumalbumin

enthält.

3. Stabilisiertes Thrombinpräparat nach Anspruch 1 oder 2, dadurch gekennzeichnet, daß es als Chelatbildner einen sechszähnigen Liganden enthält.

4. Stabilisiertes Thrombinpräparat nach Anspruch 3, dadurch gekennzeichnet, daß es als sechszähnigen Liganden Äthylendiamintetraessigsäure enthält.

5. Stabilisiertes Thrombinpräparat nach einem der vorhergehenden Ansprüche, dadurch gekennzeichnet, daß es als Proteasenhemmer Aprotinin enthält.

6. Stabilisiertes Thrombinpräparat nach einem der vorhergehenden Ansprüche, dadurch gekennzeichnet, daß es

0,03 bis 0,1 M Äthylendiamintetraessigsäure,
5 bis 20 µg/ml Aprotinin und
10 bis 40 mg/ml Serumalbumin

enthält.

## Claims

1. Stabilised thrombin preparation in solid or dissolved form, especially for analytical purposes, characterised in that, as stabiliser, it contains serum albumin in combination with at least one protease inhibitor and at least one chelate former.

2. Stabilised thrombin preparation according to claim 1, characterised in that it contains

0.01 to 0.2 M chelate former,
1 to 100 µg./ml. protease inhibitor and
5 to 100 mg./ml. serum albumin.

3. Stabilised thrombin preparation according to claim 1 or 2, characterised in that it contains a hexaligand as chelate former.

4. Stabilised thrombin preparation according to claim 3, characterised in that it contains ethylenediamine-tetraacetic acid as hexaligand.

5. Stabilised thrombin preparation according to one of the preceding claims, characterised in that it contains aprotonin as protease inhibitor.

6. Stabilised thrombin preparation according to one of the preceding claims, characterised in that it contains

0.03 to 0.1 M ethylenediamine-tetraacetic acid,
5 to 20 µg./ml. aprotonin and
10 to 40 mg./ml. serum albumin.

## Revendications

1. Préparation stabilisée de thrombine sous forme solide ou dissoute, en particulier à des fins analytiques, caractérisée en ce qu'elle contient comme stabilisants de la sérumalbumine en combinaison avec au moins un inhibiteur de protéases et au moins un agent chélatant.

2. Préparation stabilisée de thrombine suivant la revendication 1, caractérisée en ce qu'elle contient :

un agent chélatant 0,01 à 0,2 M,
1 à 100 µg/ml d'inhibiteur de protéases et
5 à 100 mg/ml de sérumalbumine.

3. Préparation stabilisée de thrombine suivant la revendication 1 ou 2, caractérisée en ce qu'elle contient comme agent chélatant un ligand hexacoordinateur.

4. Préparation stabilisée de thrombine suivant la revendication 3, caractérisée en ce qu'elle contient comme ligand hexacoordinateur de l'acide éthylènediaminetétracétique.

5. Préparation stabilisée de thrombine suivant l'une des revendications précédentes, caractérisée en

4

ce qu'elle contient comme inhibiteur de protéases de l'aprotinine.

6. Préparation stabilisée de thrombine suivant l'une des revendications précédentes, caractérisée en ce qu'elle contient :

de l'acide éthylènediaminetétracétique 0,03 à 0,1 M,
5 à 20 µg/ml d'aprotinine et
10 à 40 mg/ml de sérumalbumine.